# EUROPEAN PATENT APPLICATION

(11) **EP 4 194 054 A1**
(43) Date of publication of application: **14.06.2023**
(21) Application number: 21212985.2
(22) Date of filing: 07.12.2021
(51) Int. Cl.: A61P 31/14, C07K 16/10

(54) **CAMELID ANTIBODIES FOR USE IN THERAPY AND DIAGNOSIS**

(71) Applicant: new/era/mabs GmbH, 14482 Potsdam (DE)
(72) Inventor: HANACK, Katja, 14169 Berlin (DE); SCHLÖR, Anja, 14467 Potsdam (DE)
(74) Representative: Simandi, Claus

(57) **Abstract**

The present invention relates to compositions and methods for use of the same for the diagnosis, prevention, treatment and prophylaxis of infections caused by coronavirus. In particular the present invention relates to Camelid heavy chain antibodies and antigen binding portions thereof that specifically bind to the Spike protein of a coronavirus, in particular SARS-CoV-2 virus.

## Description

The present invention relates to compositions and methods for use of the same for the diagnosis, prevention, treatment and prophylaxis of infections caused by coronavirus. In particular the present invention relates to Camelid heavy chain antibodies and antigen binding portions thereof that specifically bind to the Spike protein of a coronavirus, in particular SARS-CoV-2 virus.

Coronaviruses (CoV) are a large family of viruses that cause illness ranging from the common cold to more severe diseases such as Middle East Respiratory Syndrome (MERS-CoV) and Severe Acute Respiratory Syndrome (SARS-CoV). A novel coronavirus (nCoV) is a new strain, recently named SARS-CoV-2 by the World Health Organization, that had not been previously identified in humans and causing a specific disease named Covid-19. Viral variants of SARS-CoV-2 are known and listed, e.g. https://covariants.org/variants/21K or https://www.gisaid.org/hcov19-variants/.

The Camelid family is composed of Camels, Alpacas and Llamas. Camelids have unique immune systems that allow them to generate immunoglobulins (antibodies) which inhibit enzymes; no other domestic animals are able to do this.

Particularly, the Camelidae produce a further subset of antibodies known as heavy chain antibodies.

These 'heavy chain' antibodies are so called as they are comprised solely of two heavy chains, wherein the light chains being present in the classical heterotetrameric antibody, are absent in the structure of the heavy chains.

The variable domain of the Camelidae heavy chain antibody differs in both structure and function to the variable heavy domain of the classical four chain antibody. Unlike the variable domain present in the heavy chain of a heterotetrameric antibody, the variable domain of the Camelidae heavy chain (referred to as VHH) has no interaction with the variable light chain domain, as this is absent. Further, the CH1 constant domain is also absent from the Camelid heavy chain antibody structure.

The variable domain (VHH) of the heavy chain contains the antigen binding sites of the antibody, which confer its binding specificity. The binding epitope of the heavy chain antibody is thus formed solely from the variable domain as opposed to being derived from a complex of the light and heavy variable domains as is the case in the classical antibody structure.

The comparative simplicity in structure, observed specific and high affinity binding to its target (paratope) confer all the advantages associated with heterotetrameric antibodies. However, heavy chain antibodies are further known to have binding specificity to enzyme active sites, and further have the ability to mimic the enzyme's substrate. Accordingly, Camelid heavy chain antibodies present advantages over classic heterotetrameric antibodies in relation to the design, production and application of clinically valuable compounds.

An outbreak of COVID-19, the disease caused by infection of the coronavirus SARS-CoV-2, began in December 2019 in China has resulted in at least two hundred million of infections and more than five million deaths up to date. Like the virus that caused the SARS outbreak several years prior, SARS-CoV, the SARS-CoV-2 virus use their spike proteins to bind host cellular receptor angiotensin-converting enzyme 2 (ACE2).

The SARS-CoV-2 spike (S) protein is a large type I transmembrane protein consisting of two subunits, S1 and S2. The S1 subunit contains a receptor binding domain (RBD) responsible for binding to the host cell receptor ACE2. The S2 subunit mediates fusion between the viral and host cell membranes.

Hence, there is a need for antibodies that bind to the spike protein and that could be used to detect and treat infection.

The present invention is directed to antigen binding domains that not only bind to the Spike protein (in short: S protein) but bind in such a way as to "lock" the Spike protein with high affinity.

The object of the present invention is therefore to provide improved and useful camelid heavy chain antibody (hcAb) or antigen binding protein or fragment thereof for the therapeutic or diagnostic use thereof for the treatment of an infection caused by a coronavirus, in particular SARS-CoV-2 virus or a SARS-CoV-2 -related disease or disorder.

SARS-CoV-2 -related disease or disorder are summarized under the term "Covid-19" and may affect the lung, heart, kidney etc. or patients may have long term covid symptoms.

The camelid heavy chain antibodies provided in accordance with the present invention are selected for their specificity to the full-length S domain (Spike protein) by phage display derived from a naive camelid library. The selected binders are produced recombinantly as camelid full-length antibodies preferably with the IgG2 isotype. Neutralization assays are performed with pseudo-viruses and SARS-CoV-2, respectively. For in vitro diagnostic applications, full length antibodies are characterized in western blot analyses, immunofluorescence and sandwich ELISAs with recombinant spike protein and SARS-CoV-2 virus like particles (VLPs).

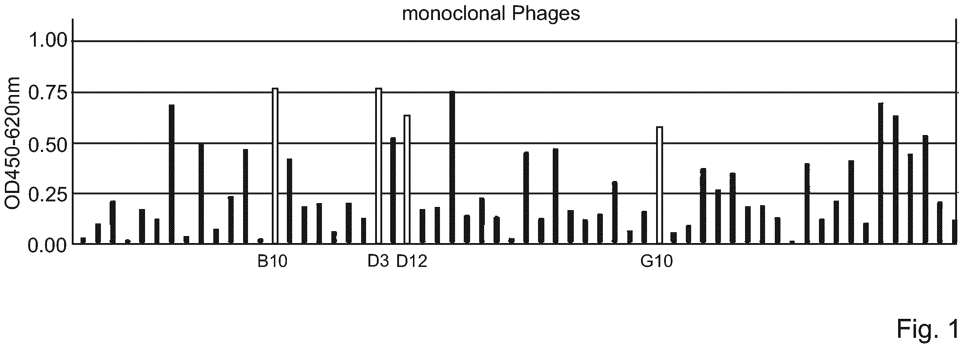

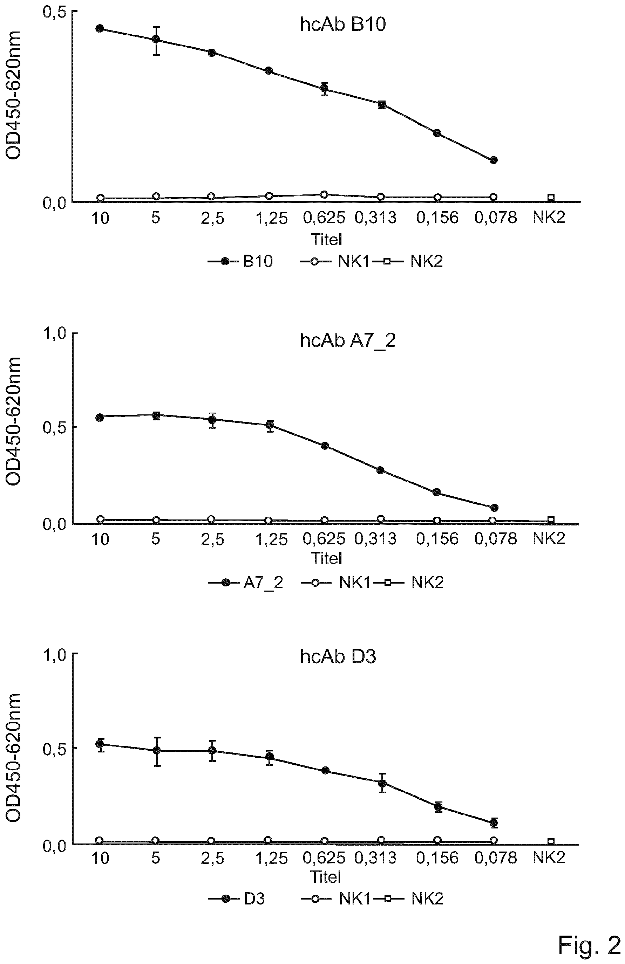

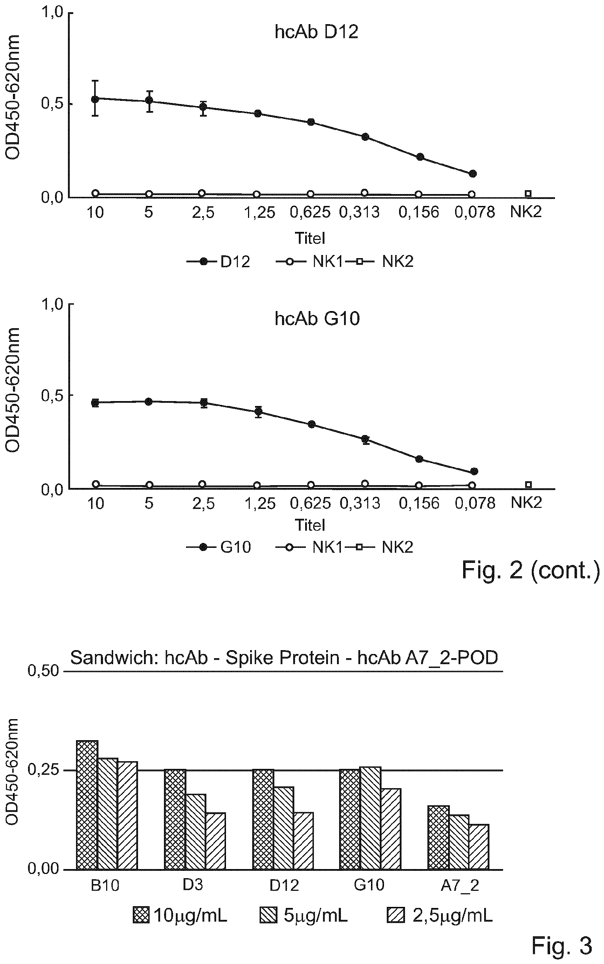

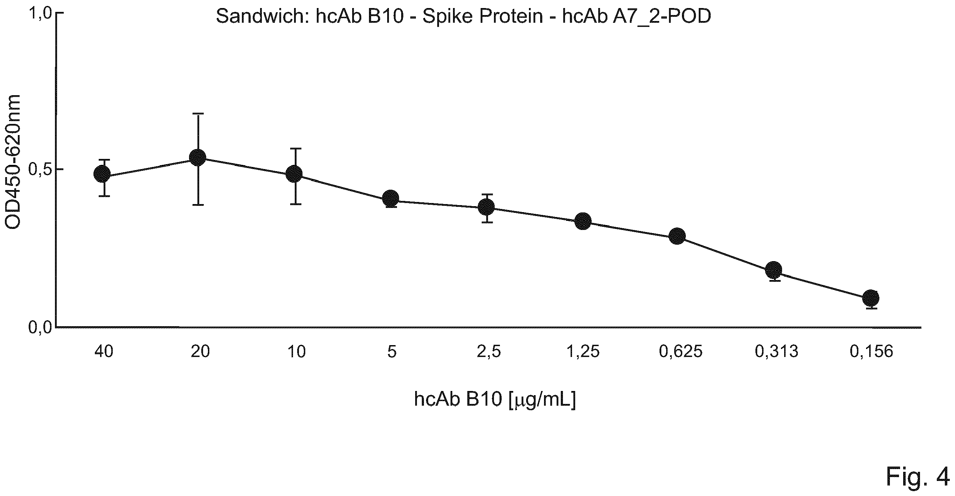

The inventors could identify more than 15 monophages with a specific ELISA signal of more than 0.5 when recombinant full length S protein was used for coating (Fig. 1). After sequencing 5 different candidates, clones A7.2, B10, D3, D12, G10 (cf. Examples, referring to SEQ ID No 1-5), are selected for recombinant expression of camelid full length IgG2 antibodies. Antibodies are purified by protein A chromatography and characterized by different ELISA formats. All antibody candidates are specifically recognizing the recombinant S protein (Spike protein). Antibody A7.2 solely recognizes the S2 subunit whereas all other candidates bind specifically to the S1 subunit. Further, the antibodies were titrated in several ELISA formats to validate their optimal concentrations for enzyme-linked immunosorbent assays (Fig. 2). Firstly, the purified full length antibody candidates are serially diluted on recombinant S protein (Fig. 2A-E) and show a specific recognition till a concentration around 0.5 µg/mL (concentration). In the next step, the inventors have investigated pairwise combinations to find the optimal catcher and detector antibodies for a sandwich immunoassay principle (Fig. 3). The usage of B10 and A7.2 is the most sensitive combination to detect recombinant full length S protein (Fig. 3). In order to provide horseradish peroxidase (HRP) based enzymatic detection, both antibody candidates are labelled successfully with said enzyme. In one preferred embodiment of the invention B10 is used as catcher and HRP-labelled A7.2 as detector (Fig. 4). The final preferred concentrations are 1 µg/mL for B10 as coating concentration and 1.25 µg/mL of HRP-labelled A7.2 as detector concentration.

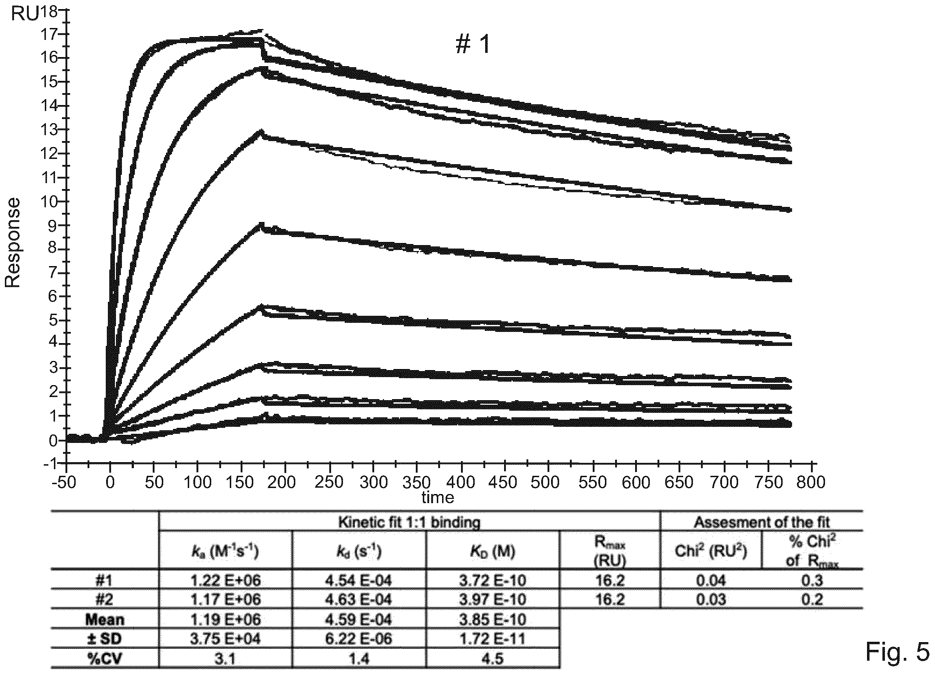

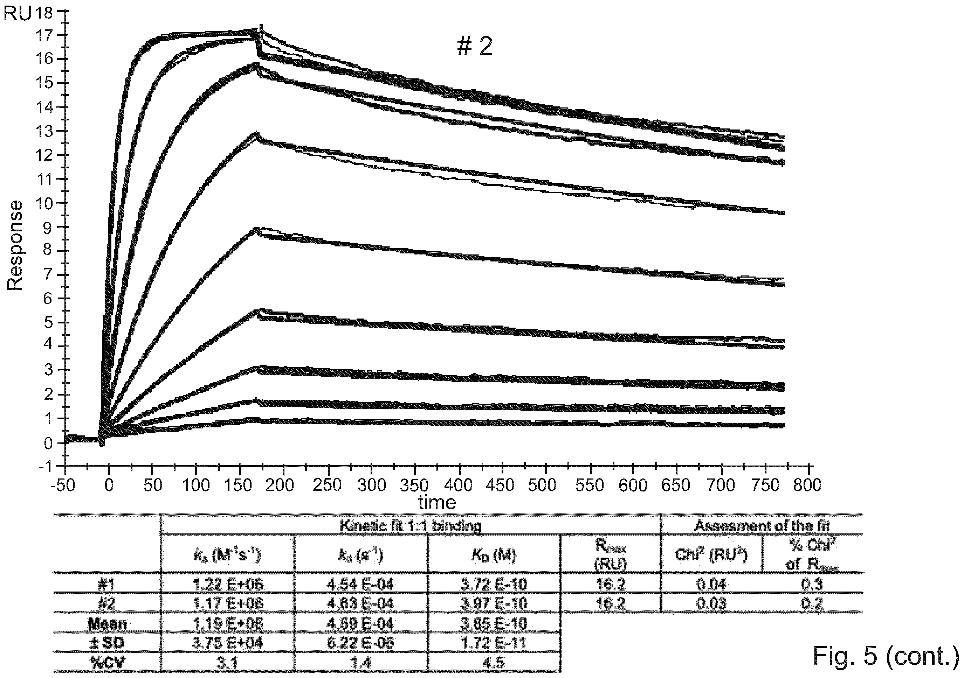

The inventors have characterized the selected antibody candidates respectively for antibody affinity and neutralization efficacy. Fig. 5 exemplarily shows the results for antibody candidate B10 in surface plasmon resonance (SPR) analysis. SPR measurements are performed with immobilized SARS-CoV-2 S protein RBD on a murine Fc. For B10 highly reproducible kinetic data could be received, revealing a K_{D} value of 0.39 nM (Fig. 5).

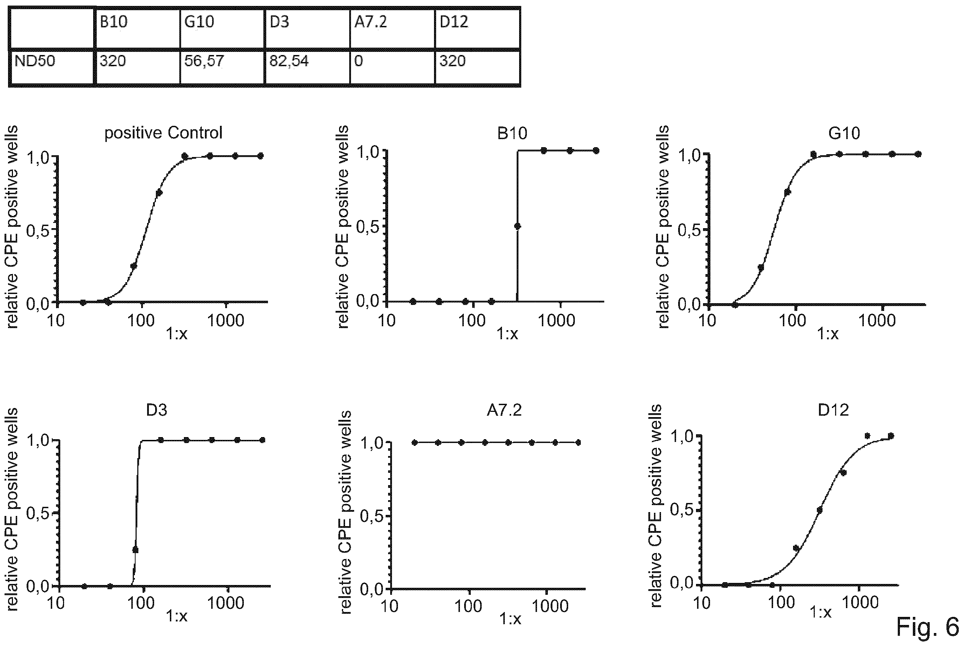

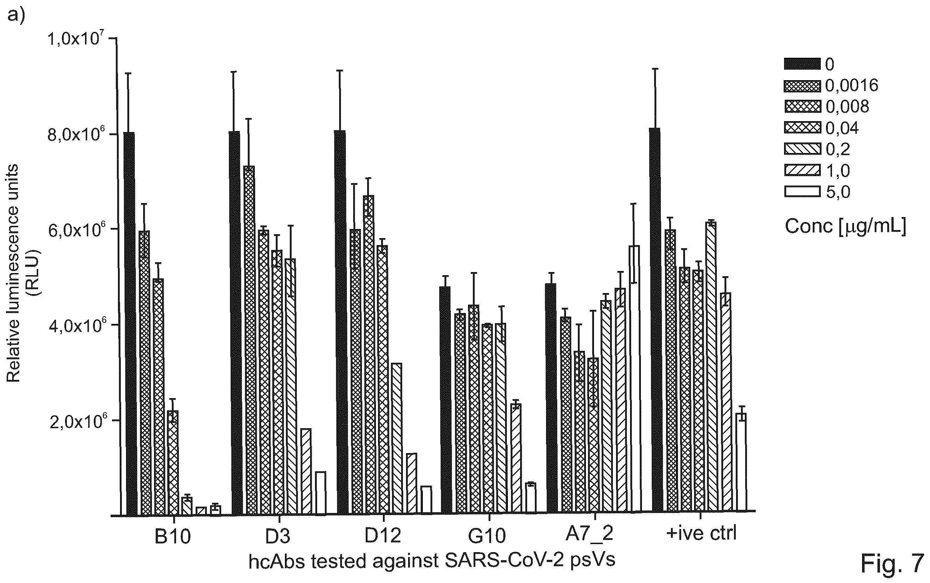

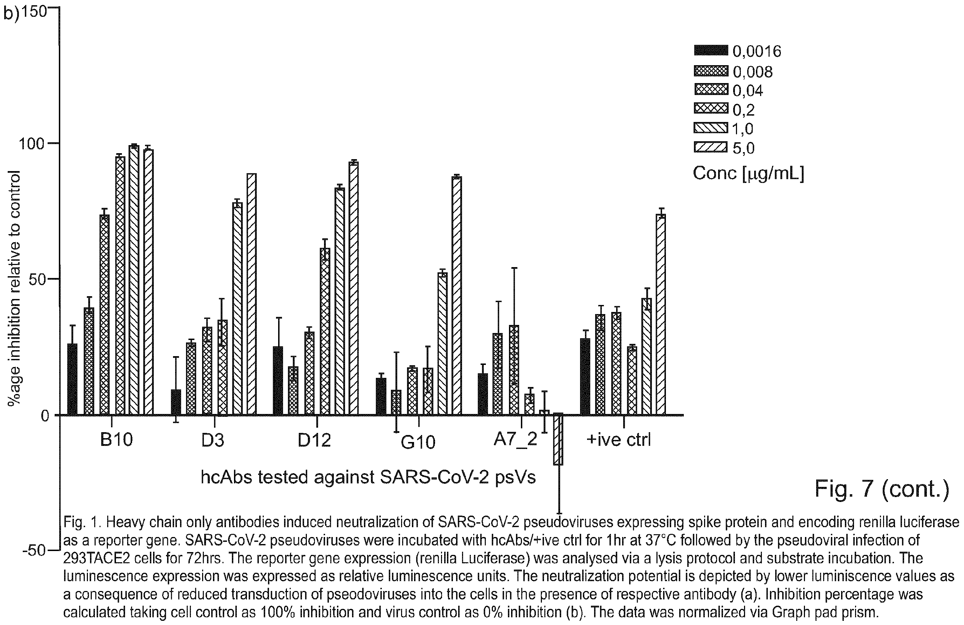

Furthermore, neutralization assays were performed with SARS-CoV-2 variants and SARS-CoV-2 pseudotyped viruses. Fig. 6 and Fig. 7 point out the results which are the same in both assays. The candidates B10, D3, D12, G10 show neutralizing capacity. B10 and D12 are strongly neutralizing 50% of the virus (ND50) at a concentration of around 1 µg/mL. D3 and G10 both showed an intermediate neutralizing activity (ND50) between 4.3-5.8 µg/mL. In pseudovirus related assays the antibody candidates showed a similar behaviour as shown in Fig. 7.

With this approach the inventors can provide efficient and specific tools useful for diagnostic applications but also neutralizing antibody candidates for therapeutic options.

Hence, the present invention refers to a camelid heavy chain antibody (hcAb) or antigen binding protein or fragment thereof comprising at least one sequence selected from the group consisting of SEQ ID No. 1 -5 or sequences thereof having an identity of at least 95%, 90%, in particular at least 80% or 70%.

In a further preferred embodiment, the invention refers to a camelid heavy chain antibody or antigen binding protein or fragment thereof comprising at least one sequence selected from the group consisting of SEQ ID No. 1 -5, wherein the antibody binds to the SARS-CoV-2 spike (S) protein.

The camelid heavy chain antibody or antigen binding protein or fragment thereof according to the invention refers to an antibody useful as a SARS-CoV-2 neutralizing antibody.

Moreover, the invention refers to a camelid heavy chain antibody or antigen binding protein or fragment thereof comprising at least one sequence selected from the group consisting of SEQ ID No. 1 -5 for use in the diagnosis, prophylaxis, prevention, and treatment of an infection caused by a coronavirus, in particular SARS-CoV-2 virus or a SARS-CoV-2 -related disease or disorder.

Hence, in a further preferred embodiment, the invention refers to a diagnostic or pharmaceutical composition comprising a camelid heavy chain antibody or antigen binding protein or fragment thereof comprising at least one sequence selected from the group consisting of SEQ ID No. 1 -5 and optional further adjuvants.

In a further embodiment of the invention, partial sequences or fragments of the sequences according to the invention are likewise comprised. In particular those partial sequences or fragments of the sequences that have an identity of at least 95%, 90%, in particular at least 80% or 70% with the sequences according to the invention.

In a preferred embodiment of the invention, the SEQ ID No. 1 - 5 refer to partial sequences or fragment comprising the binding domain or VHH or nanobody.

Moreover, in a further embodiment of the invention the camelid heavy chain antibody or antigen binding protein or fragment thereof according to the invention refer to a fusion peptide, wherein the camelid heavy chain antibody or antigen binding protein or fragment thereof according to the invention are fused with one or more polypeptides or proteins, not limited to, Fc region, hinge region, Fab region, etc. (e.g., Example 13) .

In a preferred embodiment, the determination of the heavy chain antibody or antigen binding protein or fragment thereof is carried out outside the human body and the determination is carried out in an ex vivo / in vitro diagnosis.

According to the invention, analytes are constituents of a body fluid or a discharge of a patient, i.e., a sample, in particular nasal discharge, saliva, blood, serum, urine, or of a tissue extract of the patient.

Therefore, the invention refers to a method of diagnosing a SARS-CoV-2 -related disease or disorder, comprising:
a. contacting a test sample obtained from a patient suspected of having the coronavirus-related disease or disorder with an antibody or antigen binding protein or fragment thereof according to the invention; and,
b. detecting the presence or absence of an antibody-antigen complex, wherein the presence of the antibody-antigen complex indicates that the patient has the SARS-CoV-2 -related disease or disorder.

The invention therefore likewise relates to diagnostic agents for the diagnosis of an infection caused by a coronavirus, or SARS-CoV-2 -related disease or disorder respectively antibody or antigen binding protein or fragment thereof according to the invention.

The invention therefore likewise relates to the object of providing a diagnostic device or an assay, which permits a diagnosis or examination for an infection caused by a coronavirus, in particular SARS-CoV-2 virus or a SARS-CoV-2 - related disease or disorder.

"Diagnosis" for the purposes of this invention means the positive determination of an infection caused by a coronavirus, in particular SARS-CoV-2 virus or a SARS-CoV-2 - related disease or disorder by means of the camelid heavy chain antibody or antigen binding protein or fragment thereof according to the invention as well as the assignment of the patients to infection caused by a coronavirus, in particular SARS-CoV-2 virus or a SARS-CoV-2 -related disease or disorder. The term diagnosis covers medical diagnostics and examinations in this regard, in particular in-vitro diagnostics and laboratory diagnostics.

The invention therefore relates to an assay or diagnostic device comprising an arrangement containing at least one antibody or antigen binding protein or fragment thereof according to the invention.

Within the scope of this invention, however, the term "assay" or diagnostic device likewise comprises those embodiments of a device, such as ELISA, bead-based assay, line assay, Western Blot, immunochromatographic methods, in particular immunoassays, lateral flow immunoassays, or similar immunological single or multiplex detection measures.

In another preferred embodiment of the arrangement according to the invention, the arrangement corresponds to a grid with the dimensions of a microtiter plate (8 - 12 wells strips, 96 wells, 384 wells, or more), a silica wafer, a chip, or a matrix.

The visualization of protein-protein interactions according to the invention or corresponding "means for detecting the binding success" can be performed, for example, using fluorescence labeling, biotinylation, radioisotope labeling, or colloid gold or latex particle labeling in the usual way. A detection of bound antibodies is carried out with the aid of secondary antibodies, which are labeled with commercially available reporter molecules (e.g., Cy, Alexa, Dyomics, FITC, or similar fluorescent dyes, colloidal gold or latex particles), or with reporter enzymes, such as alkaline phosphatase, horseradish peroxidase, etc., and the corresponding colorimetric, fluorescent, or chemiluminescent substrates. Readout is conducted, e.g., using a microarray laser scanner, a CCD camera, or visually.

The term "antibody" refers to a *camelid heavy chain antibody (hcAb)* presenting an immunoglobulin of any isotype, or a fragment thereof that can compete with such an antibody for specific binding to the target antigen, and includes, for instance, chimeric, humanized, bispecific antibodies. An "antibody" is a species of an antigen binding protein. An intact antibody will generally comprise at least two full-length heavy chains. The antigen binding proteins, antibodies, or binding fragments can be produced in hybridomas, by recombinant DNA techniques, or by enzymatic or chemical cleavage of intact antibodies. Camelid heavy chain antibody (hcAb) may refer to the following subclasses IgG1, IgG2 and IgG3.

The term "heavy chain" includes a full-length heavy chain and fragments thereof having sufficient variable region sequence to confer binding specificity. A full-length heavy chain includes a variable region domain, VHH. Such variable regions are called nanobodies.

The term "antigen" refers to a substance capable of inducing adaptive immune responses. Specifically, an antigen is a substance which serves as a target for the receptors of an adaptive immune response. Typically, an antigen is a molecule that binds to antigen specific receptors but cannot induce an immune response in the body by itself. Antigens are usually proteins and polysaccharides, less frequently also lipids. As used herein, antigens also include immunogens and haptens.

An "antigen binding protein or fragment thereof" ("ABP") as used herein means any protein that binds a specified target antigen. In the present invention, the specified target antigen is preferably the Coronavirus S protein or fragment thereof. "Antigen binding protein" includes but is not limited to antibodies and antigen-binding fragments thereof.

"Binding affinity" generally refers to the strength of the sum total of non-covalent interactions between a single binding site of a molecule (namely, an antibody) and its binding partner (namely, an antigen). Unless indicated otherwise, as used herein, "binding affinity" refers to intrinsic binding affinity that reflects a 1: 1 interaction between members of a binding pair, like antibody and antigen. The affinity of a molecule X for its partner Y can generally be represented by the dissociation constant (K_{d}). Affinity can be measured by common methods known in the art, including those described herein. Low-affinity antibodies generally bind antigen slowly and tend to dissociate readily, whereas high affinity antibodies generally bind antigen faster and tend to remain bound longer. A variety of methods of measuring binding affinity are known in the art, any of which can be used for purposes of the present disclosure.

By "amino acid (AA)" as used herein is meant one of the 20 naturally occurring amino acids or any non-natural analogues that may be present at a specific, defined position. By "protein" herein is meant at least two covalently attached amino acids, which includes proteins, polypeptides, oligopeptides and peptides.

The polypeptides of the invention specifically bind to the Spike trimeric protein as outlined herein. "Specific binding" or "specifically binds to" or is "specific for" a particular antigen or an epitope means binding that is measurably different from a non-specific interaction. Specific binding can be measured, for example, by determining binding of a molecule compared to binding of a control molecule, which generally is a molecule of similar structure that does not have binding activity. For example, specific binding can be determined by competition with a control molecule that is similar to the target.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope. For example, the Coronavirus S protein specific antibodies of the present disclosure are specific to SARS-CoV-2 S protein, but can cross-react with certain other Coronavirus S proteins, e.g., SARS-CoV.

The term "epitope" as used herein refers to the specific group of atoms or amino acids on an antigen to which an antibody binds. The epitope can be either a linear epitope or a conformational epitope. A linear epitope is formed by a continuous sequence of amino acids from the antigen and interacts with an antibody based on their primary structure. A conformational epitope, on the other hand, is composed of discontinuous sections of the antigen's amino acid sequence and interacts with the antibody based on the 3D structure of the antigen. In general, an epitope is approximately five or six amino acids in length. Two antibodies can bind the same epitope within an antigen if they exhibit competitive binding for the antigen.

Within the scope of this invention, "patient" means any test subject - human or mammal - with the proviso that the test subject is tested for an infection caused by a coronavirus, in particular SARS-CoV-2 virus or a SARS-CoV-2 -related disease or disorder.

In the following, the present invention is described in more detail by way of examples. However, these examples are not intended to limit the scope of protection of the present invention in any way.

### Examples

### Example 1

### Clone A7_2

AA-sequence of the hcAb-binding domain: 
DVQLQESGGGLVQPGKSLKLSCAAFGFAFSSTWMHWVRQAPGKGPEWVAFINPGGEKTMHAD
SVK GRFTISRDDAKNTLYLQMNNLKPEDTAVYYCAIERKDAFSWGQGTQVTVSS (SEQ
ID No 1)
Molecular weight: 80,1 kDa
Theoretical isoelectric point of the total hcAb: 8,1

### Example 2

### Clone B10

AA-sequence of the hcAb-binding domain: 
DVQLQESGGGLVQPGGSLRLSCAASGDINTIGTMNWFRQAPGKGREFVALITSGGTTNYADS
VKGRF TVSRDNAKNTVYLQMNSLKPEDTAVYHCYGADHYSDYWGQGTQVTVSS (SEQ ID
No 2)
Molecular weight: 79,52 kDa
Theoretical isoelectric point of the total hcAb: 6,7

### Example 3

### Clone D12

AA-sequence of the hcAb-binding domain: 
DVQLQESGGGLVQAGGSLRLSCAASGDTFLTNVMVWFRQAPGKEREFVAAIDRYGGLTRYAD
SVKG RFTISRDNDKNTLYLQMNSLKPEDTAVYYCYANRRVGGDYWGQGTQVTVSS (SEQ
ID No 3)
Molecular weight:: 80,6 kDa
Theoretical isoelectric point of the total hcAb: 7,7

### Example 4

### Clone D3

AA-sequence of the hcAb-binding domain: 
DVQLQESGGGLVQAGGSLRLSCAASGDTFLTNVMVWFRQAPGKEREFVAAIDRYGGLTRYAD
SVKG RFTISRDNDKNTNYLQMNSLKPEDTAVYYCYANRRVGGDYWGQGTQVTVSS (SEQ
ID No 4)
*D3 mit nur einem Mismatch zu D12 in FR3!
Molecular weight: 80,6
Theoretical isoelectric point of the total hcAb: 7,7

### Example 5

### Clone G10

AA-sequence of the hcAb-binding domain: 
DVQLQESGGGLVQAGGSLRLSCAASGLTSSHVYMSWFRQAPGKERDFVASILWSRGNTVYAD
SVK DRFTISRDNVKNMVYLQTNSLKPEDTAVYYCYGADHYSDYWGQGTQVTVSS (SEQ
ID No 5)
Molecular weight: 80,5 kDa
Theoretical isoelectric point of the total hcAb: 6,7

### Example 6

### Library construction and VHH-biopanning

A total of five binders (Examples 1-5) against the spike protein (SARS CoV2) were identified from a naive VHH library after five rounds of panning. The binding domains of the VHHs were fused with a camelid Fc part. The resulting heavy chain only antibodies (hcAbs) were transformed into human embryonic kidney (HEK) cells and stable cell lines were generated. By secreting the hcAbs into the culture medium, isolation from the medium is done by affinity chromatography via protein A.

A naive camelid VHH library was generated as previously described by Schlör et al (Schlör, A., Holzlöhner, P., Listek, M., GrieB, C., Butze, M., Micheel, B., Hentschel, C., Sowa, M., Roggenbuck, D., Schierack, P., Füner, J., Schliebs, E., Goihl, A., Reinhold, D., Hanack, K. (2018), Generation and validation of murine monoclonal and camelid recombinant single domain antibodies specific for human pancreatic glycoprotein 2., N Biotechnol. 2018 Apr 7. pii: S1871-6784(17)30269-8. doi: 10.1016/j.nbt.2018.03.006.). Briefly, RNA was isolated from peripheral blood mononuclear cells (PBMC; NucleoSpin RNA Mini Plus, Macherey-Nagel). For cDNA first strand synthesis RevertAid kit (Thermo Scientific) was used with a combination of random hexamer and oligo dT primers. Following a 2-step PCR protocol for VHH amplification with the primer combination P12/13 and NEM01-03_PhD. Primer sequences are listed in Table 1. Amplified VHHs were cloned into phagemid vector pADL-22c (Antibody Design Labs). The library was transformed into E. coli XL1 blue (Agilent) by electroporation.

For biopanning, transformed XL1 blue were cultured and inoculated with M13KO7 helper phages. The resulting phages were enriched in five rounds of biopanning. Full length spike protein (SPN-C52H4; AcroBiosystems) was used as antigen in decreasing amounts starting from 15 µg to 0.1 µg. Post-panning analyses were performed as described in Coomber (DOI: 10.1385/1-59259-240-6:133). Monoclonal phages were tested in phage ELISA for antigen specific binding to spike protein. Positive clones were sequenced and become heavy chain only antibodies by cloning into the expression plasmid pNEM_camAb.

### Example 7

### Heavy chain Ab construction, expression and purification

For the generation of a full length heavy chain only antibody the VHH was amplified with SL13/14 and cloned into pNEM_camhAb. pNEM_camAb is a homemade vector for eukaryotic protein expression with camelid Fc fragment (CH2-CH3 domain) under the EFlalpha promoter control resulting in a heavy chain only antibody (hcAb).

HcAbs were expressed using the Expi293 expression system (Thermo Scientific). Culturing and transfection according to the manufacturer's protocol. After 5 to 6 days after transfection, the cell culture supernatant was harvested and filtered. Followed by protein A purification (ProSep^{®} Ultra Plus; Merck). HcAbs elution was achieved by pH shift with glycine buffer as described elsewhere (Holzlöhner et al., 2018). Finally, hcAb was dialyzed against PBS (pH 7.4).

The integrity and purity of the hcAb was analyzed using SDS PAGE and ELISA.

### Example 8

### ELISA

Different ELISA formats were used.

Indirect ELISA consisted of coating microtiter plates with 0.5 - 1 µg/mL antigen, various hcAb concentrations and 1-2 µg/mL of our own HRP-conjugated mouse monoclonal anti-hcAb NEM17-11 antibody. For phage ELISA the detection of the catched phages was done with HRP-conjugated anti-M13 B62 in a dilution of 1:8.000. Sandwich ELISA was performed with hcAb in various concentrations. Each ELISA was developed with TMB substrate (Carl Roth). To block nonspecific protein binding, 1 % casein in PBS or 5 % neonatal calf serum in PBS was used.

### Example 9

### HRP Conjugation

HRP labeling of hcAb was done using a modified version of the classical periodate method [Chapter 11 Preparation of enzyme-antibody or other enzyme-macromolecule conjugates, Editor(s): P. Tijssen, Laboratory Techniques in Biochemistry and Molecular Biology, Elsevier, Volume 15, 1985, Pages 238f, ISBN 9780444806345, https://doi.org/10.1016/S0075-7535(08)70141-4].

For activation of HRP, 0.5 mg/mL HRP in 100 mM NaHCO₃ (pH 8.1) was mixed 1:1 with 12,5 mM NaIO₄ and incubated at room temperature for 2 h in the dark. In the next step same volume of activated HRP and hcAb (1 mg/mL in NaHCO₃; pH 9.2) was incubated in a glass-wool plugged Pasteur pipet filled with Sephadex G-25 (GE Healthcare) for 3 h at room temperature in the dark. Conjugated hcAb was eluated from Sephadex with 100 mM NaHCO₃ (pH 9.2). To stop the reaction, 1/20th volume of 5 mg/mL NaBH₄ was added and incubated at 4 °C. 30 min later another volume (1/10) of freshly prepared NaBH₄ solution was added and incubated at 4 °C for 1 h. Finally, after an ammonium sulfate precipitation overnight HRP conjugated hcAb was dissolved in PBS (pH 7.4).

### Example 10

### SPR

The hcAb-binding properties were analyzed at 25°C on a Biacore T200 instrument (GE Healthcare) using 10 mM HEPES pH 7.4, 300 mM NaCl, 3 mM EDTA, 0.05% Tween 20, 0.25 mg/ml BSA as running buffer. SARS-CoV-2 S protein-RBD-mFc was captured by a covalently immobilized anti-mouse IgG on a C1 sensor chip. Increasing concentrations of hcAbs (0.23 - 60 nM) were injected. Analyte responses were corrected for unspecific binding and buffer responses. Curve fitting and data analysis were performed with a kinetic fit model 1:1.

### Example 11

### Neutralisation Assay

Neutralization assays with SARS-CoV-2 wildtype and delta variant were performed as described in Zettl et.al., 2020 (Zettl F, Meister TL, Vollmer T, et al. Rapid Quantification of SARS-CoV-2-Neutralizing Antibodies Using Propagation-Defective Vesicular Stomatitis Virus Pseudotypes. Vaccines (Basel). 2020;8(3):386. Published 2020 Jul 15. doi:10.3390/vaccines8030386).

### Example 12

### Primer sequences

| Primer name | Sequence | Description |
|---|---|---|
| P12 | GTC CTG GCT GCT CTT CTA CAA GG | Primers for first round of library amplification |
| P13 | ATG GAG AGG ACG TCC TTG GGT | |
| NEM01_PhD | TCG CGG CCC AGC CGG CCA TGG CGC AGG TGC AGC TGC AGG AGT CTG GRG GAG G | Primers for second round of library amplification and cloning into pADL-22c (SfiI restriction sites) |
| NEM02_PhD | TGT TGG CCT CCC GGG CCG CTG GAG ACG GTG ACC TGG GT | |
| NEM02_PhD | TGT TGG CCT CCC GGG CCT GAG GAG ACG GTG ACC TGG GT | |
| SL13 | TTC CAC TGG TGA TGT TCA GCT GCA GGA GTC TGG GGG | Primers for cloning VHH into pNEM_camAb using InFusion (Takara) |
| SL14 | TTC CAC TGG TGA TGT TCA GCT GCA GGA GTC TGG GGG GTG TCT TGG GTT CGT TGC TGG AGA CGG TGA CCT GGG T | |

Example 13

Sequences referring to hinge and Fc-Region (CH2-CH3 domain)

## Claims

1. A camelid heavy chain antibody (hcAb) or antigen binding protein or fragment thereof comprising at least one sequence selected from the group consisting of SEQ ID No. 1 -5 or sequences thereof having an identity of at least 70%.

2. The camelid heavy chain antibody or antigen binding protein or fragment thereof according to claim 1, wherein the antibody binds to the SARS-CoV-2 spike (S) protein.

3. The camelid heavy chain antibody or antigen binding protein or fragment thereof according to claim 1 or claim 2, wherein the antibody is a SARS-CoV-2 neutralizing antibody.

4. A fusion peptide comprising a camelid heavy chain antibody or antigen binding protein or fragment thereof and one or more polypeptides or proteins.

5. The camelid heavy chain antibody or antigen binding protein or fragment thereof according to any one of the preceding claims for use in the diagnosis, prophylaxis, prevention and treatment of an infection caused by a coronavirus, in particular SARS-CoV-2 virus or a SARS-CoV-2 -related disease or disorder.

6. A diagnostic or pharmaceutical composition comprising a camelid heavy chain antibody or antigen binding protein or fragment thereof according to one of the claims 1 to 3 and optional further adjuvants.

7. A method of diagnosing a SARS-CoV-2 -related disease or disorder, comprising:
a. contacting a test sample obtained from a patient suspected of having the coronavirus-related disease or disorder with an antibody or antigen binding protein or fragment thereof of any one of claims 1-3; and,
b. detecting the presence or absence of an antibody-antigen complex, wherein the presence of the antibody-antigen complex indicates that the patient has the SARS-CoV-2 -related disease or disorder.

8. An assay or diagnostic device comprising an arrangement containing at least one antibody or antigen binding protein or fragment thereof of any one of claims 1-3.
